# EUROPEAN PATENT APPLICATION

(11) **EP 1 434 156 A2**
(43) Date of publication of application: **30.06.2004**
(21) Application number: 03024887.6
(22) Date of filing: 31.10.2003
(51) Int. Cl.: G06F 19/00

(54) **A method for conducting a clinical study**

(30) Priority: 23.12.2002 US 327787
(71) Applicant: SIEMENS AKTIENGESELLSCHAFT, 80333 München (DE)
(72) Inventor: Kuth, Rainer, 91074 Herzogenaurach (DE); Zindel, Christoph, Dr., 91080 Uttenreuth (DE)

(57) **Abstract**

In a method for conducting a clinical study, such as a clinical trial for governmental approval of a medical product, a medical facility is operated in a conventional manner over time in a number of interactions respectively with a number of patients and, for each interaction, data are produced and stored. For conducting a clinical study for a medical product that departs from the conventional interaction, an entity conducting the study identifies a need for specific data relevant to the clinical study. The stored data are made available to the entity, and the entity, or a representative of the entity, compares the stored data to the need and extracts data from the stored data to fulfill the need.

## Description

The present invention is directed to a method for conducting a clinical study for a medical product, including a method for collecting and marketing data for use in such a clinical study.

In general, a clinical study for a medical product involves the collection of data for a sufficiently large number of patients interacting with the medical product so as to permit evaluation of the efficacy and a lack of harm or harmful side effects, for the patient interacting with the medical product. As used herein, "medical product" means any item or service or procedure associated with the medical treatment or examination of a patient. Such medical products can include, but are not limited to pharmaceuticals, examination devices, treatment devices, and treatment and/or examination procedures. As used herein, a "clinical study" encompasses the type of data gathering and evaluation required for governmental approval of a particular medical product (known as clinical study), but also encompasses voluntary clinical studys which may be conducted by a manufacturer or a clinic for reasons apart from obtaining governmental approval of a particular medical product.

Medical products of all types must be tested in clinical studies in order to document their safety and effectiveness, and primarily their benefit to the patient, before being approved by governmental health authorities. A significant amount of time is required for conducting such studies, particularly for documenting the benefit of the medical product in question, because frequently the true nature of the interaction of a medical or other clinical measures with a patient can be proven only after a relatively long time. For example, a clinical measure that lengthens the life expectancy of a patient by only one week is not particularly attractive, but a clinical measure that leads to an average lengthening of life expectancy by five years is very attractive. Typically, however, the necessary proof for obtaining governmental approval for a clinical measure of the latter type would also last five years, or even longer.

There is also a demand for clinical studies to be published with a high quality level, but nevertheless in an efficient and rapid manner. Standards referred to as "normal collectives," i.e. studies that are identified and sorted according, for example, to geographical regions, can be helpful for this purpose.

It is an object of the present invention to provide a method for conducting a clinical study which assists in shortening the approval process for all types of new clinical products.

The above object is achieved in accordance with the principles of the present invention in a method for conducting a clinical study wherein patients interact with a medical facility over time during the normal course of usage of the medical facility, during which time, for each patient, data are compiled associated with the interaction of the patient with the medical facility. The compiled data are stored. In a clinical study conducted after the data are stored, for a medical product that departs from the conventional interaction with the medical facility, a need is identified by an entity conducting the clinical study for specific data relative to the clinical study. The stored data are made available to the entity, and the entity, or those under the supervision of the entity compares the stored data with the need, and extracts data from the stored data to fulfill the need. The medical facility can be of any type, such as a private practice, a clinic, a hospital, a pharmacy, a diagnostic center that offers MR, CT and/or conventional radiological examinations or any other type of medical practice that interacts with patients.

Conventionally, clinical investigations begin only when a new medical product is actually developed, and only thereafter does data compilation proceed. The inventive method departs from this conventional approach, and makes a previously compiled database available, for remuneration or other consideration, to an entity conducting a clinical study, or a representative of that entity. The already-compiled database is then compared to the need for data that is specific to the clinical study, and data which fulfill this need are extracted from the previously compiled data.

In the simplest case, for example, data arising at a diagnostic system are collected, such as during the operation of an MR scanner for a number of patients over a relatively lengthy period of time of normal operation. As is normal in the conventional operation of such a scanner, the patient name, date of birth, sex, weight, height, pathology or disease, or suspected pathology or disease, are recorded, as well as the findings made as a result of the scan and diagnosis.

Repeat examinations for the same patient can be associated with each other by being stored in linked fashion.

The patient data can be encrypted, for example with PGP so that the data associated with a particular patient can be unambiguously identified, but the identity of the patient is not revealed.

The comparison algorithm that is used to compare the stored data with the need associated with a clinical study can be of a type which can proceed even if certain data inputs are missing. Each of the above data entries, for example, can be respectively entered into a data field for storage purposes with the identity of the patient entered into the appropriate field being encrypted. If one of these fields is left blank, or if the entry therein is incorrect (for example, the date of birth) the algorithm nevertheless can make an allocation on the basis of the other data, while indicating an error probability. The error probability can be determined from demographic data, such as the frequency of occurrence of a particular entry. Data entry errors and omissions which are not of importance to the clinical study need therefore do not result in the relevant data being unusable.

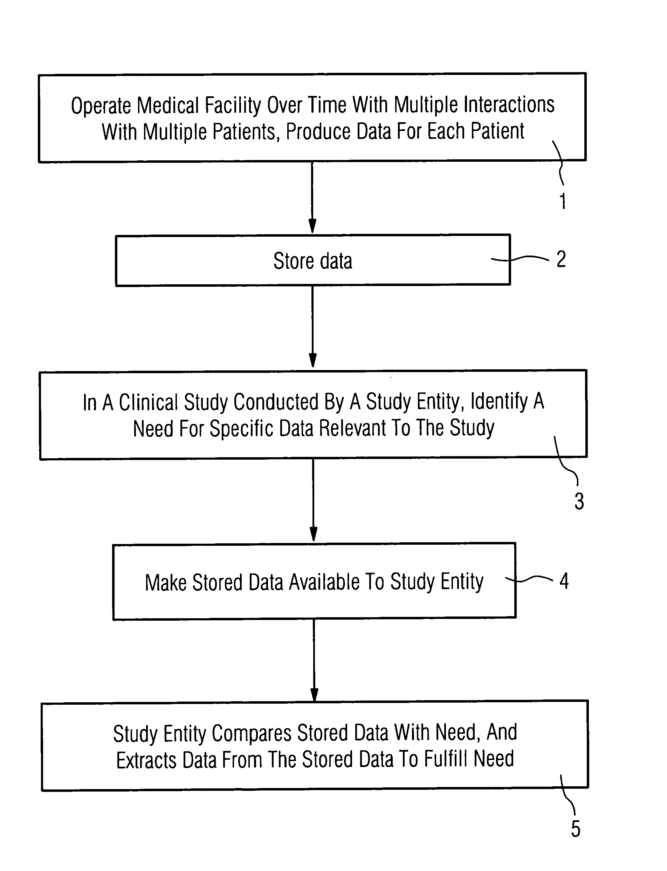

The inventive method is further described with a flowchart depicted in the accompanying figure.

The basic steps of a method for conducting a clinical study in accordance with the inventive method are shown in the figure. In step 1, a medical facility is operated in the normal course of business over time, during which a number of patients interact with the medical facility. For each patient, data relating to the interaction are acquired. The acquired data are stored in step 2.

In step 3, for a clinical study conducted by an evaluation entity for a product that departs from the conventional interaction in step 1, a need for specific data relating to the clinical study is identified. In the fourth step, the data stored in step 2 are made available to the entity, or a representative of the entity. In step 5, the entity or the entity's representative compares the stored data to the need identified in step 3, and data are extracted from the stored data to fulfill the need.

As noted above, in step 1, the acquired data can be appropriately encrypted to protect the privacy of the patient, and stored in step 2 in such an encrypted manner.

An example of the manner by which the inventive method can be employed is for a clinical study of a new contrast agent that concentrates twice as quickly in a low-grade (benign) glioma as compared to currently available, conventional contrast agents, as would be employed in the day-to-day use of an MR scanner as the medical facility in step 1. The likelihood that such a low-grade glioma would be diagnosed early would be enhanced by the new contrast agent. The "need" in step 3 of the inventive method in this example would be a need for data regarding patients in whom a low-grade glioma was suspected, but was not found in initial examinations, but was later found to be present. If an adequately large statistical ensemble exists in the data compiled and stored in steps 1 and 2, a conclusion can be made as to how much earlier a diagnosis would have been possible with the new contrast agent, and what therapy options would then have been available at an earlier stage.

Another example is the evaluation of a new imaging method. Such an imaging method could be a quantitative method wherein specific data are acquired and evaluated (for example, bone density). Such data must be compared to a normal collective (standard) such as a geographically specific normal collective. The need in this case is therefore not only defined as a medical need, but also as a geographic need in step 3.

In each of the above examples, the availability of a pre-existing database, from which data can be extracted to fulfill the need arising in the clinical study significantly accelerates the overall testing and evaluation process. Making the stored database available for ultimate use in the clinical study, in step 4, can proceed in a number of different ways.

In one embodiment, owners of the medical entity that acquires or collects the data can also store the data and make the data available to suitable entities, such as pharmaceutical companies, to implement the clinical study. Alternatively, the stored data can be offered for sale to any qualified customer, on an exclusive or non-exclusive basis.

In another embodiment, the owner of the medical entity can transfer the data, with suitable protection to avoid revealing patient identities, to a commercial databank operator, who then collects similar data from many medical facilities and makes the overall database available in one of the ways described above. The data suppliers, i.e., the medical facilities, can either be paid on a transaction basis or can share in the income resulting from making the data available for clinical studies, either on a commission basis or as a percentage of the profits of the databank operator.

In a further embodiment, the manufacturer of a clinical system, sold to and then used by the medical facility, can provide consideration to the medical facility in return for the medical facility making the data available to the manufacturer. Such consideration can be in the form of price discounts, rebates, or other commercial inducements. In another embodiment, the data are stored in a data carrier that physically belongs to the patient, or are stored in a manner by which the patient remains the owner of the data. If stored in a data carrier that belongs to the patient, the data carrier should be of a type which only allows successive entries to be made, with no deletions or alterations. New data then can be added to the database in the data carrier, but stored data cannot be deleted or altered. The patient can then sell the stored data to a databank operator either outright, or as a copy with the patient still retaining the data. Data carriers suitable for this purpose are currently commercially available in the form in multi-session mode CD-ROMs. Other suitable types of storage media for this purpose are PROMs, for example, in the form of a flat chip on a plastic card, such as in the form of a debit card. This embodiment has the advantage that a patient having a complex illness, i.e., an illness for which prognosis and/or pathology are still relatively unknown from a scientific or clinical point of view, can systematically make data available upon each follow-up interaction with the medical facility so that progress, or the lack thereof, can be well documented.

The inventive method can also be used to allow the entity to identify suitable patients for a particular clinical study. The approval process for a new medication currently typically costs between approximately 50 and 100 million dollars (with an equivalent cost in euros). Pharmaceutical manufacturers, for example, can make a commitment to such an investment only when a certain degree of exclusivity, such as by filing for and/or obtaining patents, for the particular medication exists. The commercialization, and thus the recovery of the development costs, for the finished medical product therefore must ensure in a time window between obtaining approval to sell the pharmaceutical, which conventionally may occur years after the application for or grant of a patent, and the expiration of the patent or patents covering the product. Pharmaceutical manufacturers therefore have a significant desire to keep the time necessary to obtain approval to sell the product as short as possible. Clinical studies are a significant part of the approval process for new pharmaceuticals. Such studies require the identification and selection of a large number of patients having a specific illness for which the pharmaceutical is intended, as well as patients have appropriate epidemiological parameters (for example, age, weight, sex, etc.). During the course of the clinical study, one or more clinics must test the effect of the pharmaceutical as well as the side effect thereof, and compare and document these test results to conventional pharmaceuticals. This procedure generally has a cost associated therewith of approximately $5,000 per patient. The selection of suitable patients for such a study is therefore expensive and complicated. Clinics are inclined to select patients for the study from the limited number of their own patients, even if those patients may not be ideally or optimally suited for the study, because many activities of a clinic, particularly research projects, are paid for from the income derived from clinical studies. The communication between the pharmaceutical manufacturer and the clinic or clinics, moreover, may be complicated by a dependency relationship. The clinic would like to place as many of its own patients as possible in the study, with as small as possible an outlay on the part of the clinic. The pharmaceutical manufacturer is primarily interested in obtaining a maximum quality of the study as well as a fast implementation of the study, and therefore would like to exclude patients from the study who are not ideally suited therefore. Compromises or shortcuts may result from this tension, which may ultimately degrade the quality of the study.

Magnetic resonance scanner manufacturers generally do not participate in the clinical evaluation of a new pharmaceutical. The number of such scanner manufacturers in the world is relatively small. For example, three manufacturers of magnetic resonance scanners (GE, Philips and Siemens) control approximately 85% of the world market. The scanner manufacturers are neutral with respect to the numerous pharmaceutical manufacturers. At the same time, however, such scanner manufacturers respectively have contractual relationships with a large number of clinics. Each manufacturer typically has a contractual relationship with approximately 30% of the total number of clinics which employ this type of equipment. For example, it is estimated that more than 50,000 persons are examined daily using all scanner manufactured by Siemens. In the case of a pharmaceutical clinical study for a contrast agent, for example, data thus are available for qualification or identification of patients for the study at least in a time window form the registration of a patient until the findings which are made using the scanner. As of the moment, such data are not systematically collected (even though the data may be archived), and in particular are not inter-clinically compiled nor made available to pharmaceutical manufacturers. Conventionally, a pharmaceutical manufacturer conducting a clinical study for a new contrast agent must attempt to recruit one or more clinics for the study who can make patients available for that purpose. This can result in limitations in the study quality, such as being limited to a relatively low number of patients.

In the case of a magnetic resonance scanner, or any other type of imaging or diagnostic system, a dedicated software module can be added to the operating software for compiling the data used in the inventive method. The compiled data can include, but is not limited to, a code for the clinic that will store the data in readable form with identification of the patient, consecutive numbering of the patients in the clinic. data of birth, sex, body weight, suspicion or reason for the examination, and a findings report. The data optionally also can include genetically relevant data such as specific risk factors insofar as they are known, the cause of death of parents, grandparents and siblings, blood type, other illnesses experienced by the patient, images (particularly those most important for the diagnosis) and other measured data such as, for example, MR spectra.

The data are individually (i.e., per patient) communicated in encrypted form to a databank, such as a databank maintained by a databank operator, in which case the communication can proceed by e-mail. The data are then entered into and stored in the databank. The databank operator can then make a commercial offer to a pharmaceutical company to search for patients having specific features for a particular clinical study in which the pharmaceutical company is interested. The operator communicates the "hits" (without revealing the identity of the patient, since this is unknown to the databank operator) to the pharmaceutical manufacturer. The pharmaceutical manufacturer can then contact the clinic directly, and the clinic can then determine whether to participate in the study and how to go about obtaining patient approval for participation in the study.

Alternatively, the databank operator may have a contract with many clinics and a secured data exchange communication link with each clinic. In the simplest case, the interest of a clinic is to be a participant in as many clinical studies as is reasonable. The databank operator, however, may be a joint venture of several or all-participating clinics. The quality of the findings report in the communicated data, as to accuracy, level of detail and understandability, influences the likelihood that a particular clinic will be able to contribute patients for a study, this being a source of income for that clinic. The patient also profits therefrom, even if no direct remuneration is paid to the patient, since the patient's participation in the clinical study may result in the patient's illness being more effectively treated as a result of the clinical study. The pharmaceutical company obtains the advantage of not being limited to one or a few clinics in the selection of patients for a study, and can also make, or have made for it, a pre-evaluation of the quality of the data.

The databank operator thus functions as a broker and can make an extremely large number of patient available for a particular study, such as one million or more patients.

Alternatively, a pharmaceutical company may open an account with the databank operator into which data are entered in return for payment, and the pharmaceutical company can do its own searching.

The databank can either be operated by, or associated with, a scanner pharmaceutical, so that the scanner manufacturer can offer special conditions or rebates associated with the sale of scanners to induce a clinic to provide data to the databank operator. Alternatively, the scanner manufacturer may contract operation of the databank to an outside entity.

The data compiled in this manner in accordance with the invention can be used for purposes other than conducting clinical studies. The data can be made available, for remuneration or otherwise, to professional associations, such as the AMA, for evaluating pharmaceuticals and therapies for particular illnesses. Committees of such professional associations may be confronted with the task of prioritizing particular clinical studies or other medical research. For example, very expensive measures may lead to only to a comparatively slight increase in the health or quality of life a patient, whereas other inexpensive measures may produce a pronounced enhancement. The cost associated with a particular medical measure can be identified from the aforementioned data, by identifying the frequency and number of follow-up examinations for the same patient, and the results (i.e improvement or no improvement) documented for each examination.

A pharmaceutical manufacturer, for example, may consult a database compiled in accordance with the inventive method for making decisions regarding development of a particular product, i.e. for assessing the potential market for a new product before beginning development thereof, and without the necessity of having to begin to compile "new" data, which thereby lengthens the decision-making process.
Other information, if recorded in the acquired data, can be used for a variety of purposes, such as the typical (average) examination duration, use of resources (contrast agent, sedatives, anesthetics, etc.), use of personnel, etc. Although modifications and changes may be suggested by those skilled in the art, it is the intention of the inventors to embody within the patent warranted hereon all changes and modifications as reasonably and properly come within the scope of their contribution to the art.

## Claims

1. A method for conducting a clinical study comprising the steps of:
(a) operating a medical facility over time in a plurality of conventional interactions respectively with a plurality of patients and, for each of said interactions, producing data;
(b) storing said data as stored data;.
(c) in a clinical study conducted by an evaluation entity for a medical product that departs from said conventional interaction, identifying a need for specific data relevant to said clinical study;
(d) making said stored data available to said entity; and
(e) said entity comparing said stored data to said need and extracting data from said stored data to fulfill said need.

2. A method as claimed in claim 1 wherein step (a) includes producing data including data representing an identity of each patient, and wherein step (b) comprises encrypting at least said data representing the identity of each patient.

3. A method as claimed in claim 1 or claim 2 wherein step (d) comprises making said stored data available to said entity directly from said medical facility.

4. A method as claimed in one of the claims 1 to 3 wherein step (d) comprises communicating said stored data from said medical facility to a databank operator, and making said stored data available to said entity via said databank operator.

5. A method as claimed in one of the claims 1 to 4 wherein step (b) comprises for each patient, providing a storage medium in which data produced by interaction of that patient with said medical facility are stored, and wherein step (d) comprises making said stored data available to said entity from said storage medium directly from each patient.

6. A method as claimed in one of the claims 1 to 5 wherein said medical facility is a clinic which operates an imaging device, sold by an imaging device manufacturer, and wherein said interactions comprise respective examinations of patients with said imaging device.

7. A method as claimed in claim 6 comprising the additional step of establishing a contractual relationship between said imaging device manufacturer and said entity, and wherein step (d) comprises making said stored data available to said entity dependent on said contractual relationship.

8. A method as claimed in claim 6 or 7 comprising providing consideration from said imaging device manufacturer to said clinic upon a sale of said imaging device from said imaging device manufacturer to said clinic in return for said clinic making said stored data available to said entity in step (d).

9. A method as claimed in claim 8 wherein said entity is a pharmaceutical manufacturer and wherein said examination includes administration of a contrast agent and wherein step (c) comprises identifying a need for specific data relating to diagnosis of patients using said contrast agent.

10. A method as claimed in one of the claims 1 to 9 wherein step (a) comprises producing said data for each of said interactions with said respective patient selected from the group consisting of, for each patient, a patient identifier, date of birth, sex, body weight, reason for the interaction, and findings resulting from the interaction.

11. A method as claimed in one of the claims 1 to 12 wherein step (a) comprises producing said data for each of said interactions with said respective patient selected from the group consisting of, for each patient, a patient identifier, date of birth, sex, body weight, reason for the interaction, findings resulting from the interaction, genetic data, cause of death of close relatives, blood type, other illnesses, medical images, and other measured data.
